# EUROPEAN PATENT SPECIFICATION

(11) **EP 4 070 828 B1**
(45) Date of publication and mention of the grant of the patent: **16.10.2024**
(21) Application number: 22167142.3
(22) Date of filing: 07.04.2022
(51) Int. Cl.: A61M 5/168, A61M 5/142, A61M 39/22

(54) **FLUID DELIVERY PUMP VALVE ASSEMBLY**
FLUIDFÖRDERPUMPENVENTILANORDNUNG
ENSEMBLE SOUPAPE DE POMPE D'ADMINISTRATION DE FLUIDE

(30) Priority: 07.04.2021 US 202163171813 P
(43) Date of publication of application: 12.10.2022
(73) Proprietor: Insulet Corporation, Acton MA 01720 (US)
(72) Inventor: KURZMAN, Maya, Marlboro (US); CARDINALI, Steven, Tewksbury (US); BREINGAN, Kyle, Acton (US)
(74) Representative: Peterreins Schley

(56) References cited:
- US-A1- 2020 338 262

## Description

### FIELD OF THE INVENTION

The present disclosure generally relates to a pump for infusing a patient with a fluid, for example, a pump arranged within a wearable medicament delivery system, and, in particular, a multi-state valve for controlling the flow of the fluid through the pump.

### BACKGROUND

Healthcare providers may prescribe patients wearable devices for delivering fluids, such as liquid medicaments, as part of a treatment regimen. Non-limiting examples of medicaments may include chemotherapy drugs, hormones (for instance, insulin), pain relief medications, and other types of liquid-based drugs. In general, wearable medicament delivery devices are relatively small form factors that may be adhered to the skin of the patient, with a reservoir to hold the medicament. The device may include a needle or cannula fluidically coupled to the reservoir and extending from the device and into the skin of the patient. A pump may operate to force the fluid from the reservoir, through a fluid path, and out through the needle and into the patient (as e.g. disclosed in US 2020/0338262 A1). A control system, with hardware and/or software elements, may be arranged within the device to manage medicament delivery and other device features. The control system may operate alone or in combination with an external computing device, such as a patient smartphone, healthcare provider computer, and/or the like.

Minimizing the footprint of a wearable medicament delivery device makes the device less obtrusive to the patient and improves the overall user experience. Accordingly, developers are consistently under pressure to reduce the size of operating components, while increasing the features (for example, monitoring patient physiological information, increasing automation, and/or the like) while limiting power demands. In order to achieve a smaller and more compact device, components are required to be smaller and more compact. For example, current pumping mechanisms include a valve switch to allow a user to fill the reservoir and to dispense a medicament. The valve must be shut while the user fills the reservoir, and open when the pump begins working. The valve must also differentiate between filling and dispensing of the pump. This entails three states for the valve, for example, closed, open for filling, and open for dispensing. Accordingly, conventional pump systems are not able to efficiently and effectively manage fluid flow within a device while meeting the space and energy demands of devices that are desired by patients.

Therefore, there is a need for an improved pumping mechanism for a wearable medicament delivery device that can be made compact and energy efficient while achieving a desired dosage accuracy.

### SUMMARY

This Summary is provided to introduce a selection of concepts in a simplified form that are further described below in the Detailed Description.

The present invention is directed to a fluid pump system for a wearable fluid delivery device as defined in claim 1.

In some embodiments of the fluid pump system, the wearable fluid delivery device may be configured to deliver a medicament to a patient, the medicament comprising insulin. In some embodiments of the fluid pump, the movable portion may include a bistable mechanism.

In exemplary embodiments of the fluid pump system, the state of the fluid path may include one of a fluid delivery state to deliver the fluid to the patient from the pump chamber or a fluid fill state to fill the pump chamber from a reservoir in fluid communication with the fluid path.

In various embodiments of the fluid pump system, the actuator may include a shape memory alloy. In some embodiments of the fluid pump, the offset may include a substantially triangular projection extending from the movable portion. In various embodiments of the fluid pump system, the fluid pump may include a spring configured to bias the shaft in a direction toward the movable portion in the absence of the force of the actuator. In exemplary embodiments of the fluid pump system, the shaft may include a detent arm.

In some embodiments of the fluid pump system, the movable portion may be associated with a first magnet attracted to a portion of the fixed portion to maintain the movable portion in contact with the fixed portion on a closed side of the fluid path valve until the shaft engages the movable portion to pivot the movable portion. In various embodiments of the fluid pump system, the V-feature may include at least one channel for shaft to travel during operation of the fluid path valve. In some embodiments of the fluid pump system, the at least one channel may include a sloped channel with at least one drop-off element to prevent the shaft from engaging an inner wall of the movable portion when traveling toward a central V-section of the V-feature.

### BRIEF DESCRIPTION OF THE DRAWINGS

**FIG. 1** illustrates an exemplary operating environment in accordance with the present disclosure;
**FIG. 2** illustrates an exemplary wearable fluid delivery device in accordance with the present disclosure;
**FIG. 3** illustrates an embodiment of a fluid delivery pump in accordance with the present disclosure;
**FIGS. 4A** and **4B** illustrate an embodiment of a fluid delivery pump in accordance with the present disclosure;
**FIG. 5** illustrates an embodiment of a fluid path valve in accordance with the present disclosure;
**FIGS. 6A-6E** illustrate operation states of an embodiment of a fluid delivery pump in accordance with the present disclosure;
**FIG. 7** illustrates an embodiment of a fluid path valve in accordance with the present disclosure;
**FIG. 8** illustrates an embodiment of a fluid path valve in accordance with the present disclosure;
**FIG. 9** illustrates an embodiment of a fluid path valve in accordance with the present disclosure; and
**FIG. 10** illustrates an embodiment of a fluid path valve in accordance with the present disclosure.

The drawings are not necessarily to scale. The drawings are merely representations, not intended to portray specific parameters of the disclosure. The drawings are intended to depict example embodiments of the disclosure, and therefore should not be considered as limiting in scope. In the drawings, like numbering represents like elements

### DETAILED DESCRIPTION

The described technology generally relates to a wearable fluid delivery device for delivering a fluid to a patient. In some embodiments, the fluid may be or may include a medicament. The wearable fluid delivery device may include a reservoir for holding the fluid, a fluid path in fluid communication with the reservoir, a needle in fluid communication with the fluid path to deliver the fluid to the patient wearing the wearable fluid delivery device, and a fluid delivery pump configured to force the fluid from the reservoir, through the fluid delivery path, and into the patient via the needle.

In some embodiments, a fluid path valve or switch may be within, or in fluid communication with, the fluid path. The fluid path valve may be configured to alternate between multiple states or positions to configure the flow of the fluid through the fluid path. For example, operation of the fluid delivery pump may be configured to alternate between filling a pump chamber with fluid from the reservoir and pumping the fluid from the pump chamber into the patient. The fluid path valve may be set into a first state for filling the pump chamber and a second state to allow operation of the pump to move the fluid from the pump chamber and into the patient. In various embodiments, the fluid path valve may be a bistable valve having two stable positions. In exemplary embodiments, the fluid path valve may be or may include a "push-push" mechanism configured to actuate between at least two states. In various embodiments, the fluid path valve may be or may include a bistable push-push mechanism. In general, bistable push-push mechanism may enter two possible states, alternating between each state with each "push" or actuation. For example, a bistable push-push mechanism may enter a first state responsive to a first actuation, a second actuation may cause the bistable push-push mechanism to enter a second state, a third actuation may cause the bistable push-push mechanism to re-enter the first state, a fourth actuation may cause the bistable push-push mechanism to re-enter the second state, and so on.

In a conventional fluid delivery device, a pump, such as a reciprocating pump, may require a fluid path that alternates between filling a pump chamber with fluid from a reservoir, and pumping fluid from the chamber into the patient. One example technique to accomplish this is by incorporating an independent actuator to alternate the system between these two states, which would decrease the required load on the pumping actuators. A shape-memory alloy (SMA) wire or other actuator may be used to drive the system; however, one actuator is only able to control one direction of movement in the system, which would be ineffective or greatly increase energy consumption if the actuator needed to hold the fluid path in one position.

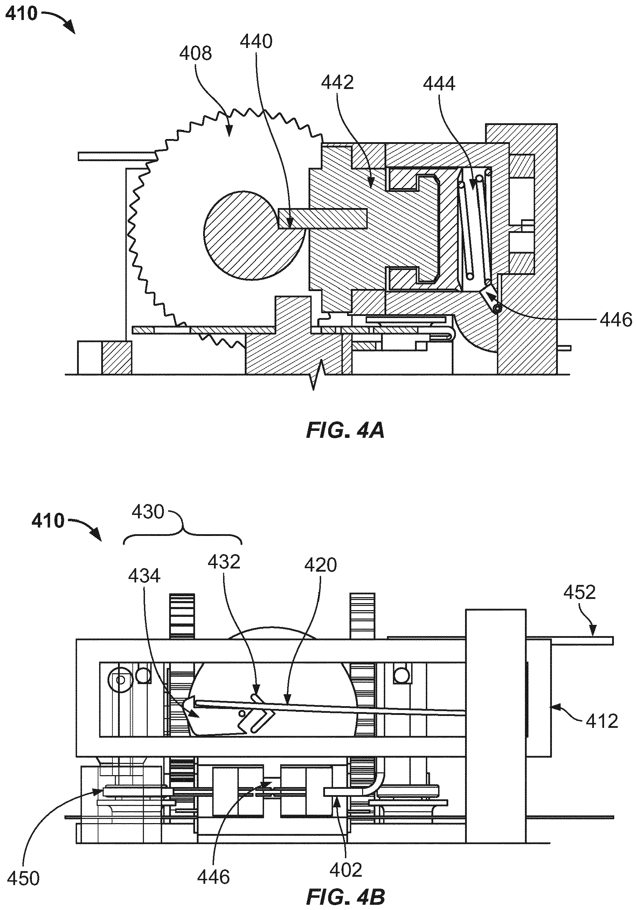

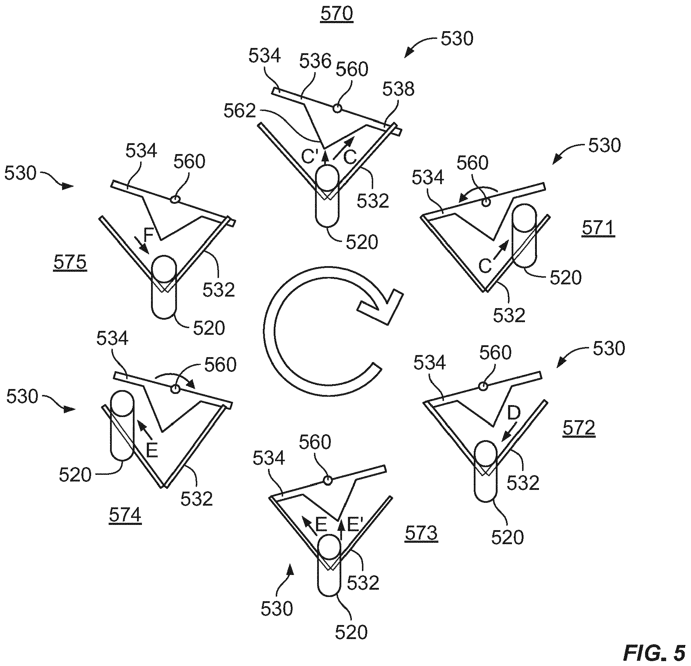

Accordingly, a mechanism to reliably alternate between two or more positions is required. In some embodiments, for example, a detent (or detent arm, arm, rod, shaft, pin, rod, push-push element, and/or the like) actuated by an actuator (for example, via an SMA wire and/or a spring) may be placed inside a fluid pathway (for instance, as a fluid pathway valve) that can alternate between two configurations, and attaching them to the needle in the pump, the system may reliably rest in two positions (for instance, a fluid fill state and a fluid delivery state). In some embodiments, the general shape of the path may be or may substantially be a "V" shape, for example, where the inside will be attached to a bistable mechanism that is fixed at the center (see, for example, FIGS. 4B and 5). In various embodiments, an inner corner of the wall of the path may be offset from the outer corner, which may change based on the configuration of the bistable mechanism. By offsetting the corner of the inner wall, the detent may reliably move to one side of the V-shaped path. The configuration of the bistability may be altered when the detent contacts it and causes it to deflect at each of the top points of the V-shaped path (see, for example, FIG. 5).

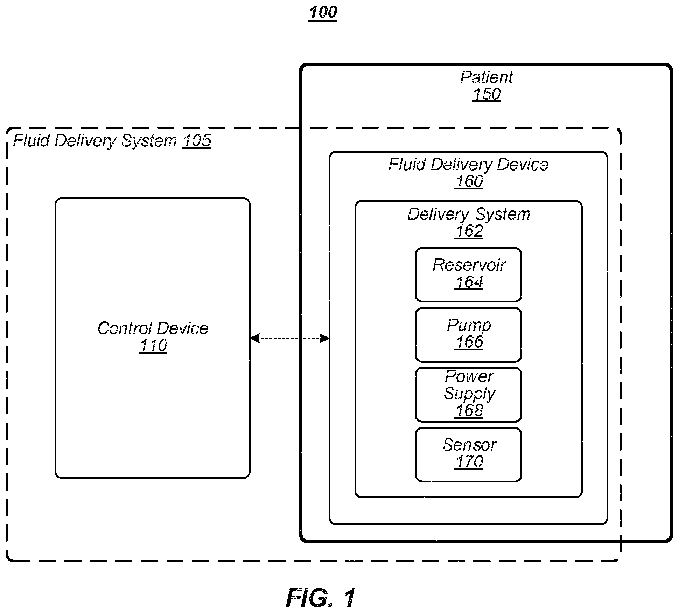

**FIG. 1** illustrates an example of an operating environment 100 that may be representative of some embodiments. As shown in FIG. 1, operating environment 100 may include a fluid delivery system 105. In various embodiments, fluid delivery system 105 may include a control or computing device 110 that, in some embodiments, may be communicatively coupled to a fluid delivery device 160. Computing device 110 may be or may include one or more logic devices, including, without limitation, a server computer, a client computing device, a personal computer (PC), a workstation, a laptop, a notebook computer, a smart phone, a tablet computing device, a personal diabetes management (PDM) device, and/or the like. Embodiments are not limited in this context.

Fluid delivery device 160 may be or may include a wearable automatic fluid delivery device directly coupled to patient 150, for example, directly attached to the skin of the user via an adhesive and/or other attachment component.

In some embodiments, fluid delivery device 160 may be or may include a medicament delivery device configured to deliver a liquid medicament, drug, therapeutic agent, or other medical fluid to a patient. Non-limiting examples of medicaments may include insulin, GLP-1, pramlintide, glucagon, pain relief drugs, hormones, blood pressure medicines, morphine, methadone, chemotherapy drugs, proteins, antibodies, a co-formulation of more than one of the foregoing, and/or the like.

In some embodiments, fluid delivery device 160 may be or may include an automatic insulin delivery (AID) device configured to deliver insulin (and/or other medication) to patient 150. For example, fluid delivery device 160 may be or may include a device the same or similar to an OmniPod^{®} device or system provided by Insulet Corporation of Acton, Massachusetts, United States, for example, as described in United States Patent Nos. 7,303,549; 7, 137,964; and/or 6,740,059. Although an AID device and insulin are used in examples in the present disclosure, embodiments are not so limited, as fluid delivery device 160 may be or may include a device capable of storing and delivering any fluid therapeutic agent, drug, medicine, hormone, protein, antibody, and/or the like.

Fluid delivery device 160 may include a delivery system 162 having a number of components to facilitate automated delivery of a fluid to patient 150, including, without limitation, a reservoir 164 for storing the fluid, a pump 166 for transferring the fluid from reservoir 164, through a fluid path or conduit, and into the body of patient 150, and/or a power supply 168. Fluid delivery device 160 may include at least one penetration element (not shown) configured to be inserted into the skin of the patient to operate as a conduit between reservoir 164 and patient 150. For example, penetration element may include a cannula and/or a needle. Embodiments are not limited in this context, for example, as delivery system 162 may include more or fewer components.

In some embodiments, fluid delivery device 160 may include at least one sensor 170 operative to detect, measure, or otherwise determine various physiological characteristics of patient 150. For example, sensor 170 may be or may include a continuous glucose monitoring (CGM) sensor operative to determine blood glucose measurement values of patient 150. In another example, sensor may be or may include a heart rate sensor, temperature sensor, and/or the like.

In various embodiments, fluid delivery device 160 may be a closed-loop fluid delivery system using sensor 170 as an internal monitor to track patient information, either directly or via control device 110, to delivery system 162 to formulate delivery of a fluid to patient 150 via the penetration element.

In some embodiments, computing device 110 may be a smart phone, PDM, or other mobile computing form factor in wired or wireless communication with fluid delivery device 160. For example, computing device 110 and Fluid delivery device 160 may communicate via various wireless protocols, including, without limitation, Wi-Fi (i.e., IEEE 802.11), radio frequency (RF), Bluetooth^{™}, Zigbee^{™}, near field communication (NFC), Medical Implantable Communications Service (MICS), and/or the like. In another example, computing device 110 and fluid delivery device 160 may communicate via various wired protocols, including, without limitation, universal serial bus (USB), Lightning, serial, and/or the like. Although computing device 110 (and components thereof) and fluid delivery device 160 are depicted as separate devices, embodiments are not so limited. For example, in some embodiments, computing device 110 and fluid delivery device 160 may be a single device. In another example, some or all of the components of computing device 110 may be included in fluid delivery device 160. For example, Fluid delivery device 160 may include processor circuitry, memory unit, and/or the like. In some embodiments, each of computing device 110 and fluid delivery device 160 may include a separate processor circuitry, memory unit, and/or the like capable of facilitating insulin infusion processes according to some embodiments, either individually or in operative combination. Embodiments are not limited in this context.

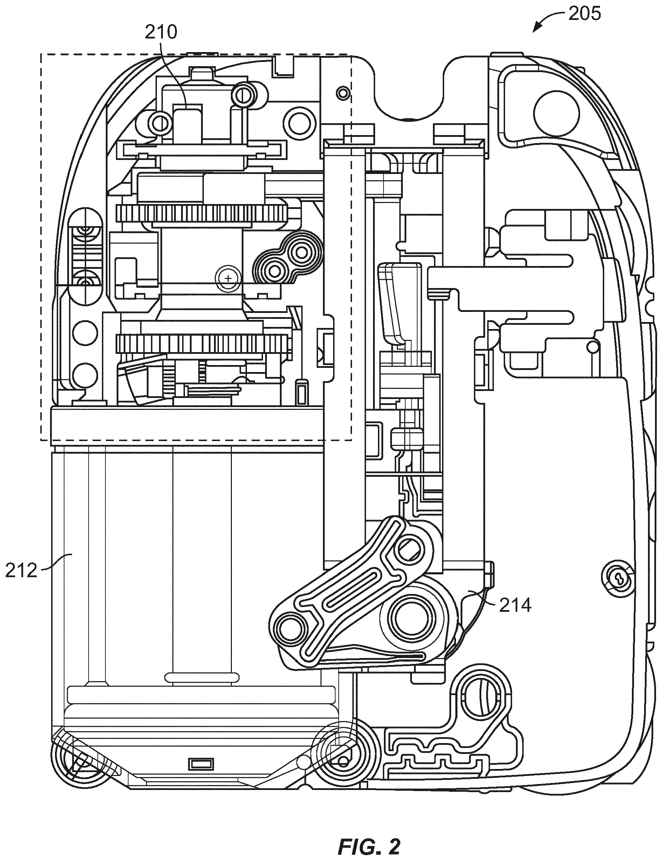

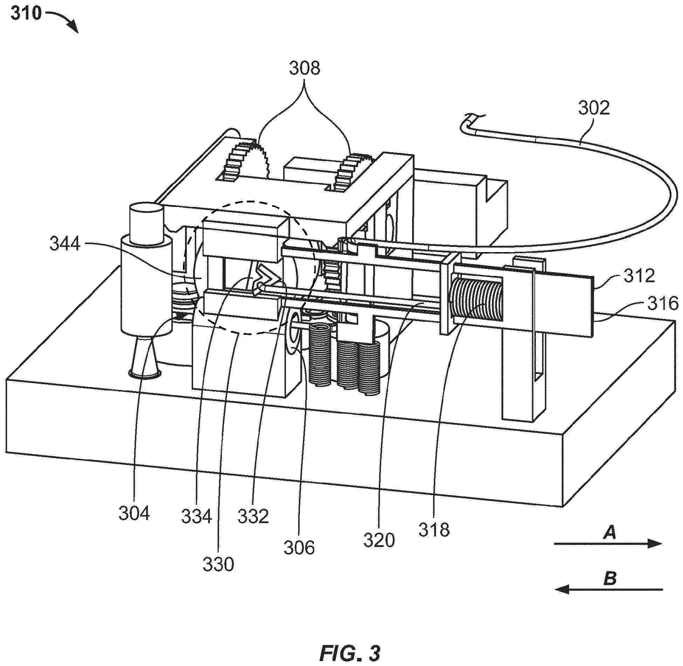

**FIG. 2** illustrates an exemplary wearable fluid delivery device in accordance with the present disclosure. In particular, FIG. 2 depicts a top-down view of a wearable fluid delivery device 205. As shown in FIG. 2, a wearable fluid delivery device 205 may include multiple systems to store and delivery a fluid to a patient. Fluid delivery device 205 may include one or more housings configured to enclose the multiple systems. In some embodiments, wearable fluid delivery device 205 may include a pump 210. In various embodiments, pump 210 may be or may include a reciprocating pump (see, for example, FIGS. 3, 4A, and 4B). In exemplary embodiments, wearable fluid delivery device 205 may include a reservoir 212 for storing a fluid. Reservoir may be in fluid communication with pump 210 for delivering the fluid to patient via needle 214.

In various embodiments, pump 210 may be a multi-dose reciprocating pump. In some embodiments, pump 210 may be configured to deliver about 0.25 microliters per pulse. In exemplary embodiments, pump 210 may have a footprint of about 23 millimeters (mm) × 28 mm × 12 mm.

**FIG. 3** illustrates an embodiment of a fluid delivery pump in accordance with the present disclosure. As shown in FIG. 3, a fluid delivery pump 310 may include a ratchet or ratchet wheels 308 operably coupled to a snail cam (not shown, see, for example, FIG. 4A), a reservoir inlet 304 fluidically coupled to a reservoir (not shown), and a patient outlet 306 fluidically coupled to a needle 302. Rotation of ratchet wheels 308 may cause fluid to flow from the reservoir to a pump chamber 344 or through needle 302 to patient depending on a flow path state, for instance, whether the pump is in a chamber fill state (fluid fill state) or a patient infusion state (fluid delivery state), respectively.

A fluid path valve 330 may be configured to switch the flow path state of pump 310 from a first state (for instance, a fluid fill state or chamber fill state) to a second state (for instance, a fluid delivery state or patient infusion state).

In some embodiments, fluid path valve 330 may include a fixed portion 332 and a movable portion 334. In some embodiments, fixed portion 332 may be an outer wall of fluid path valve 330. In various embodiments, fixed portion 332 may be a V-feature. In exemplary embodiments, movable portion 334 may pivot about a pivot point. In various embodiments, movable portion 334 may be a pivotable inner wall of fluid path valve 330. In some embodiments, movable portion 334 may be or may include a bistable push-push mechanism (push-push mechanism, bistable mechanism, or inner wall).

A detent (or shaft, rod, pin, and/or the like) 320 may be configured to engage V-feature 332 and/or bistable mechanism 334 to set the state of fluid path valve 330. In some embodiments, detent 320 may be coupled to a bracket 312 or other structure. In various embodiments, forces intended to move detent 320 may be wholly or partially imparted on bracket 312 to move detent 320. In other embodiments, forces intended to move detent 320 may be wholly or partially imparted directly on detent 320.

Although element 334 is described in examples in the present disclosure as a bistable mechanism, push-push mechanism, or bistable push-push mechanism, embodiments are not so limited. For example, element 334 may be or may include other mechanisms besides a bistable mechanism, push-push mechanism, or bistable push-push mechanism. In another example, element 334 is not limited to a bistable mechanism, push-push mechanism, or bistable push-push mechanism or may be a push-push mechanism with more than two states. Rather, use of examples of a bistable mechanism, push-push mechanism, or bistable push-push mechanism are intended to provide illustrative functional embodiments of element 334. Accordingly, element 334 may be or may include any mechanism, element, structure, and/or the like capable of operating according to some embodiments.

Although element 332 is described in examples in the present disclosure as a V-feature, embodiments are not so limited. For example, element 332 is not required to be "V" shaped. Rather, use of a "V" shape or the term V-feature are not intended to be limiting but are intended to provide illustrative functional embodiments of element 332. Accordingly, element 334 may be or may include any mechanism, element, structure, and/or the like, with various different shapes, capable of operating according to some embodiments.

In some embodiments, forces intended to move detent 320 may be wholly or partially imparted on bracket 312 to move detent 320 may be actuated or moved by an actuator 316. In various embodiments, actuator may be or may include an SMA wire operably coupled to detent 320 and/or a bracket 312. In exemplary embodiments, a spring 318 may be operably coupled to detent 320 and/or a bracket 312. In some embodiments, actuator 316 may impart a force on detent 320 to move detent in direction A (for instance, away from bistable mechanism 334). In some embodiments, actuator 316 may impart a force on detent 320 to move detent in direction B (for instance, toward bistable mechanism 334).

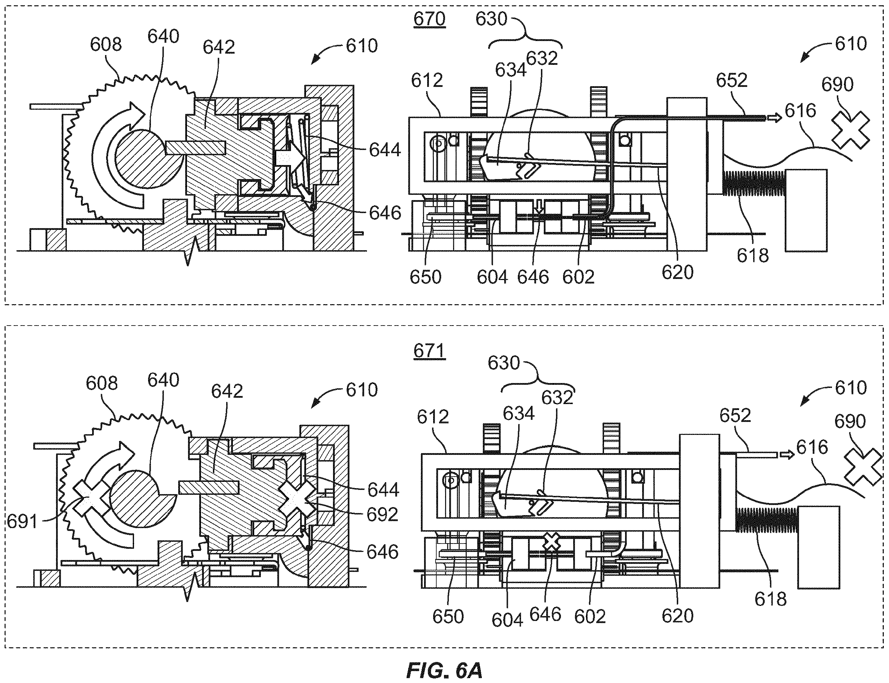

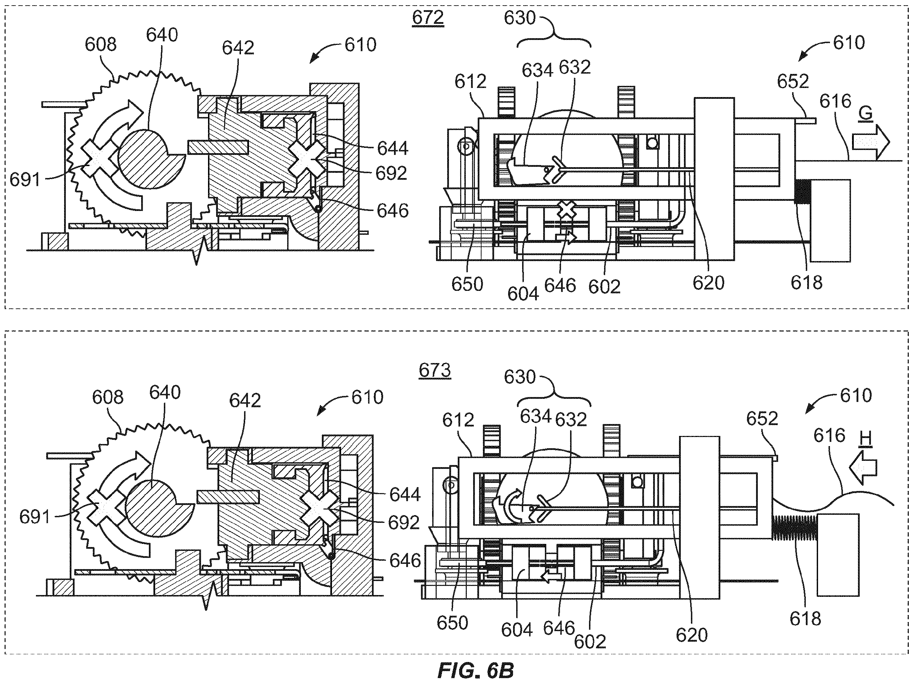

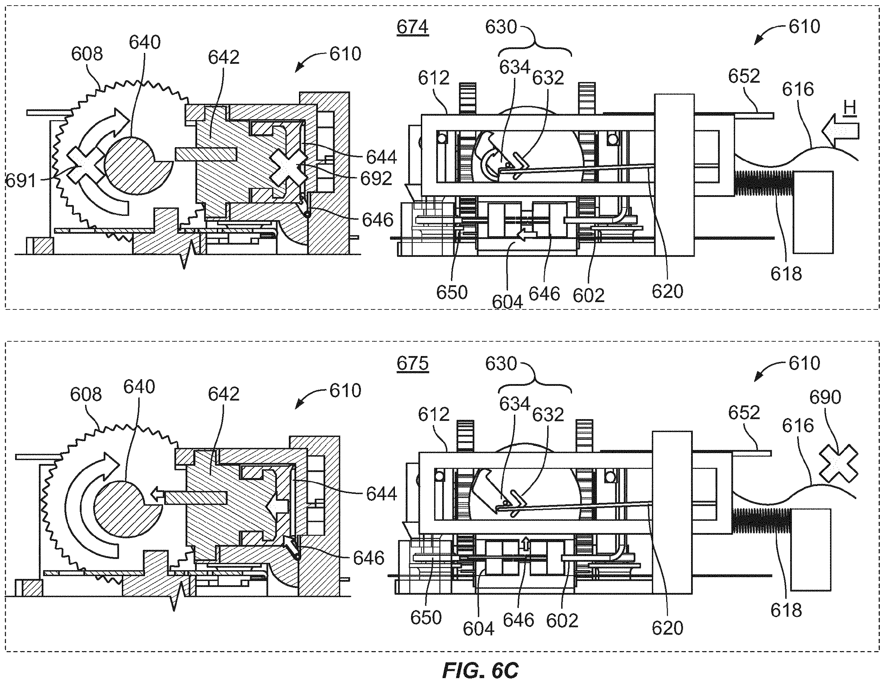

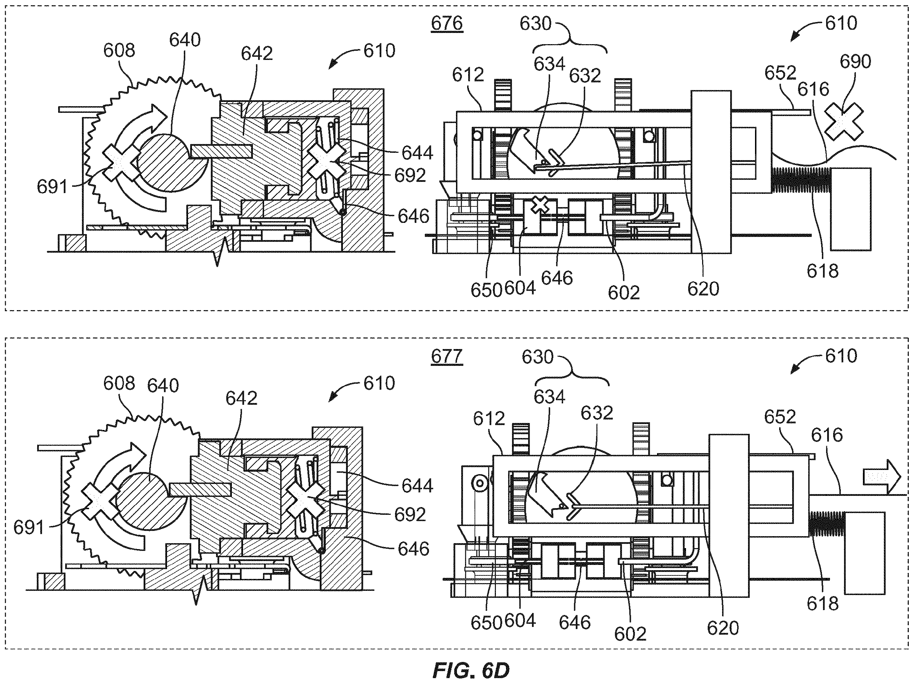

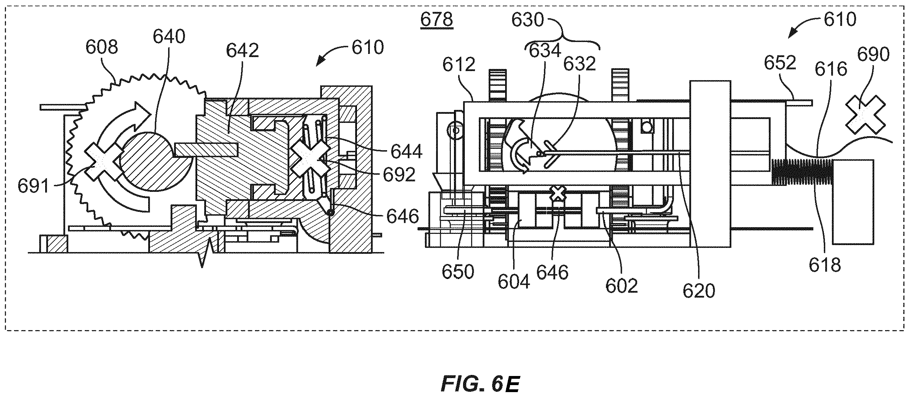

In some embodiments, a spring 318 may operate to bias detent 320, for example, in the direction of arrow B. Accordingly, if actuator 316 is not providing a force to move detent 320 in the direction of arrow A, detent 320 may be biased in the direction of arrow B. For example, an SMA wire actuator 316 may be activated to move detent 320 in the direction of arrow A. Relaxation of SMA wire actuator 316 may cause detent 320 to move back in the direction of arrow B. In this manner, fluid path valve 330 may be switched between fluid path states (see, for example, FIGS. 6A-6E).

In some embodiments, actuator 316, and other components of pump 310, may be actuated via hardware and/or software of a wearable fluid delivery device and/or an external computing device communicatively coupled to wearable fluid delivery device (see, for example, FIG. 1).

**FIGS. 4A** and **4B** illustrate an embodiment of a fluid delivery pump in accordance with the present disclosure. More specifically, FIGS. 4A and 4B show a cross-sectional side view and a front view, respectively, of a fluid delivery pump 410. In some embodiments, fluid delivery pump 410 may be a reciprocating pump.

As shown in FIGS. 4A and 4B, pump 410 may include a ratchet wheel 408, a snail cam 440, a piston 442, and a chamber 444. In some embodiments, a fluid path of pump 410 may include a reservoir entrance to needle 450, a chamber/needle passthrough 446, and a needle 402 with a needle exit 452. A fluid path valve 430 may include a V-feature 432 and a bistable mechanism 434.

During operation of pump 410, fluid path valve 430 may be placed in a chamber fill state, for example in which a reservoir (not shown) is open to chamber 444 and closed to needle 402 (and patient). Rotation of ratchet 408 may rotate past the drop off of snail cam 440 and piston 442 may reset to chamber 444. Fluid path valve 430 may be switched to the patient infusion state, for example, in which chamber inlet 450 is closed to reservoir and needle 402 is open to chamber 444. In the patient infusion state, rotation of ratchet 408 may cause fluid to be pumped from chamber 444, through needle 402, and into the patient.

**FIG. 5** illustrates an embodiment of a fluid path valve in accordance with the present disclosure. More specifically, FIG. 5 depicts steps 570-575 of fluid path valve 530 as fluid path valve 530 transitions between states. As shown in FIG. 5, fluid path valve 530 may include a fixed portion (or V-feature) 532 and a movable portion (or bistable mechanism) 534 operably coupled to a pivot element 560 configured to allow bistable mechanism 534 to pivot responsive to force provided by a shaft or detent 520. In some embodiments, V-feature 532 and bistable mechanism 534 may form a path, for example, with V-feature 532 being configured as a fixed outer wall of the path and bistable mechanism 534 being an inner wall of the path (that pivots on pivot element 560).

In some embodiments, V-feature 532 may include a path, groove, inset, slot, wall, or other structure that may guide detent 520, for example, responsive to forces imparted by an actuator and/or spring. In various embodiments, V-feature 532 may be offset or non-parallel and/or may include projections to cause detent 520 to deflect in a desired direction. In exemplary embodiments, bistable mechanism 534 may have an offset or projection 562 (for instance a tip of a triangular or substantially triangular portion) that may be offset to cause detent 520 to deflect in a desired direction. Projection 562 may form a first side, inner wall, extension, overhang, arm, or other structure 536 of bistable mechanism on a first side of projection 562 and a second side, inner wall, extension, overhang, arm, or other structure 538 on a second side of projection (first wall 536 and second wall 538 are only labeled in step 570 to limit the complexity of FIG. 5). Projection 562 may be offset to cause detent 520 to deflect in a desired direction to engage first wall 536 or second wall 538 to cause bistable mechanism to tilt, pivot, or otherwise move in a desired direction to change a state of fluid path valve 530.

At step 570, fluid path valve 530 may be in a first state (for example, a chamber fill state). Detent may be pulled in direction C' at step 570, for example, in response to an actuation by an SMA wire or other actuator (or conversely, due to the force of a biasing spring in the absence of actuation by an SMA wire). However, offset 562 (for instance, a triangular projection) of bistable mechanism 534 may be configured to cause detent 520 to deflect in direction C (for instance, to the right) and contact second wall 538. For example, as detent 520 is pulled up toward bistable mechanism, detent 520 may hit at or near the point of the triangular projection of offset 562, causing detent 520 to veer into direction C.

At step 571, detent 520 may contact bistable mechanism 534 on the right side (for instance, at second wall 538), causing bistable mechanism 534 to rotate counterclockwise. Fluid delivery valve may now be in a second state, such as a patient infusion state, after rotating to create an opening between V-feature 534 and second wall 538. At step 572, detent 520 may get pulled in direction D and come to a rest in a corner of V-feature 534.

At step 573, detent may be pulled in direction E', however, at this stage, the orientation of offset 562 of bistable mechanism 534 may cause detent 520 to deflect in direction E (for instance, to the left). At step 574, detent 520 may contact bistable mechanism 534 on the left side (for instance, at first wall 536), cause bistable mechanism 534 to rotate clockwise. At step 575, detent 520 may get pulled in direction F and come to a rest in a corner of V-feature 534.

Process of steps 570-575 may be repeated to switch the flow path from a first state (for instance, a chamber fill state) to a second state (for instance, a patient infusion state). For example, steps 571-573, in which V-feature 534 is open on the upper right side, may be a first state, and steps 570, 574, and 575, in which V-feature 534 is open on the upper left side may be a second state, or vice versa.

**FIGS. 6A-6E** illustrate operation states of an embodiment of a fluid delivery pump in accordance with the present disclosure. More specifically, FIGS. 6A-6E depict steps 670-678 of a fluid infusion process (for instance, including chamber fill and patient infusion processes), depicting a cross-sectional side view and a front view for a pump 610 according to some embodiments.

As shown in FIG. 6A, a pump 610 may include a ratchet 608, snail cam 640, piston 642 and chamber 644. In some embodiments, a fluid path of pump 610 may include a reservoir entrance to needle 650, a chamber/needle passthrough 646, and a needle 602 with a needle exit 652. A fluid path valve 630 may include a V-feature 632 and a bistable mechanism 634.

In some embodiments, a detent 620 may be operably coupled to a bracket 312/612. An actuator 616 may be operably coupled to bracket 312 to impart a force on detent 620. In some embodiments, actuator 616 may be or may include an SMA wire that, when activated, may pull detent 620. In exemplary embodiments, a spring 318/618 may be operably coupled to detent 320/620 and/or a bracket 312/612 to bias detent 320/620 (for instance, in the absence of a force by actuator 616).

At step 670, the fluid path is in a first position (for instance, a patient infusion position) such that rotation of ratchet wheel 608 may pump the fluid out of chamber 644 and into the patient. This infusion pulse may be repeated for multiple pulses, such as about 50 to about 100 pulses. Actuator 616 is deactivated, as indicated by X 690. At step 671, ratchet wheel 608 may stop (indicated by X 691 as the drop off of snail cam 640 approaches. In step 671, pump 610 may stop moving the fluid (as indicated by X 692) and the fluid path remains in the patient infusion position.

At step 672, SMA 616 is activated, pulling detent 620 in direction G. Detent 620 may center on V-feature 632 and the fluid path may move out of the patient infusion position. At step 673, SMA 616 is deactivated, initiating SMA release, causing detent 620 to move in direction H. Detent 620 may contact and cause rotation (for instance, clockwise rotation) of bistable mechanism 634. The fluid path may move into a second position (for instance, a chamber fill position).

At step 674, SMA release continues such that detent 620 continues moving in direction H. Detent 620 further rotates bistable mechanism 634 and moves the fluid path into the chamber fill position. At step 675, ratchet wheel 608 may rotate and piston 642 may fall off of the drop off of snail cam 640, creating a vacuum or negative pressure in chamber 644 that draws fluid from the reservoir, through 650 and into chamber 644.

At step 676, ratchet wheel 608 may stop moving and chamber 644 may be at least partially full of the fluid. The fluid path may be in a chamber fill (or non-infusion) position. At block 677, SMA 616 may be activated, pulling detent 620 in direction H. Detent 620 may center on V-feature 632 and the fluid path may move out of the chamber fill position. At step 678, SMA 616 may be deactivated and detent 620 may contact and rotate (for instance, counterclockwise) bistable mechanism 634. The fluid path may be moved into the patient infusion position.

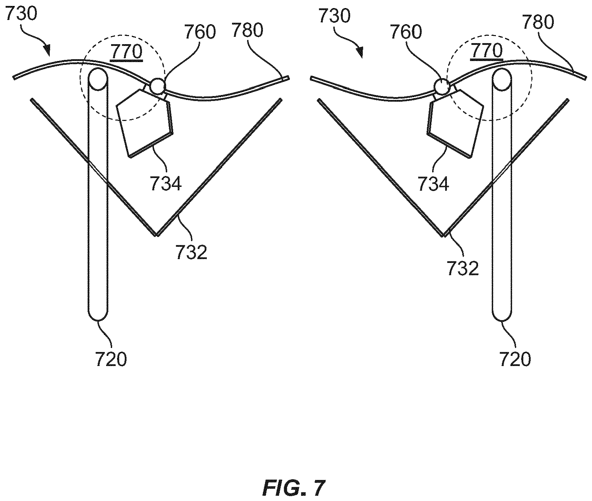

**FIG. 7** illustrates an embodiment of a fluid path valve in accordance with the present disclosure. As shown in FIG. 7, a fluid path valve 730 may include a V-feature 732 and a bistable mechanism 734. In some embodiments, bistable mechanism 734 may include a beam 780. In various embodiments, a shape of bistable mechanism 734 may be configured to reduce or even eliminate detent 720 from being caught, stuck, or otherwise trapped, either temporarily or permanently, in area 770 between bistable mechanism 734 and beam 780. This embodiment may be used in the system shown in earlier figures.

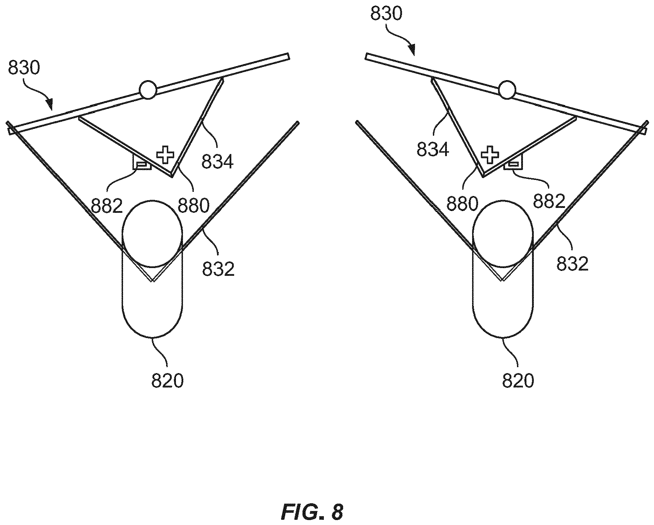

**FIG. 8** illustrates an embodiment of a fluid path valve in accordance with the present disclosure. As shown in FIG. 8, a fluid path valve 830 may include a V-feature 832 and a bistable mechanism 834. In some embodiments, bistable mechanism 834 may be associated with magnets. For example, a bottom face of path 882 and an inside wall 880 may have opposite magnetic charges. Accordingly, when detent 820 reaches the top of the path and pushes inside wall 880 over center, the opposite poles may operate to facilitate complete motion and prevent bistable mechanism 834 from passing back over center as detent 820 returns to the bottom (or corner) of V-feature 832. For example, the magnets may operate to maintain bistable mechanism 834 in contact with V-feature on a closed side of fluid path valve 830 until detent 820 engages bistable mechanism 834 to pivot bistable mechanism 834 (i.e., to prevent bistable mechanism 834 from swinging back after detent 820 moves toward the V-section (inner corner) of V-feature 832). This embodiment may be used in the system shown in earlier figures. Additionally or alternatively, there may be sufficient friction in the fluid path valve 830 such that when the bistable mechanism 834 rotates to one side, the friction in the fluid path valve 830 prevents the bistable mechanism 834 from rotating back to the center or toward the other side, until it is contacted again by detent 820.

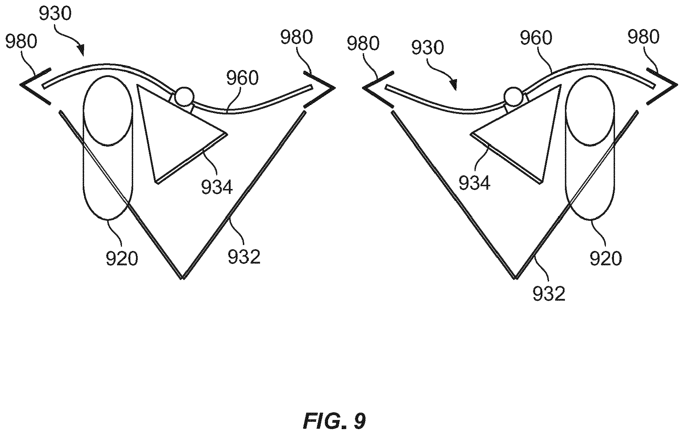

**FIG. 9** illustrates an embodiment of a fluid path valve in accordance with the present disclosure. As shown in FIG. 9, a fluid path valve 930 may include a V-feature 932 and a bistable mechanism 934. In some embodiments, a beam 960 may be fixed at the center where it is connected to the inner walls of the path, constrained on either side 980 of fluid path valve 930.

When detent 920 reaches a top of the path, detent 920 may cause the bends of beam 860 to invert, pushing the inner wall of bistable mechanism 934 over center. The bending forces and stresses of beam 960 may facilitate complete motion and prevent bistable mechanism 934 from passing back over center as detent 920 returns to the bottom (or corner) of V-feature 932. This embodiment may be used in the system shown in earlier figures.

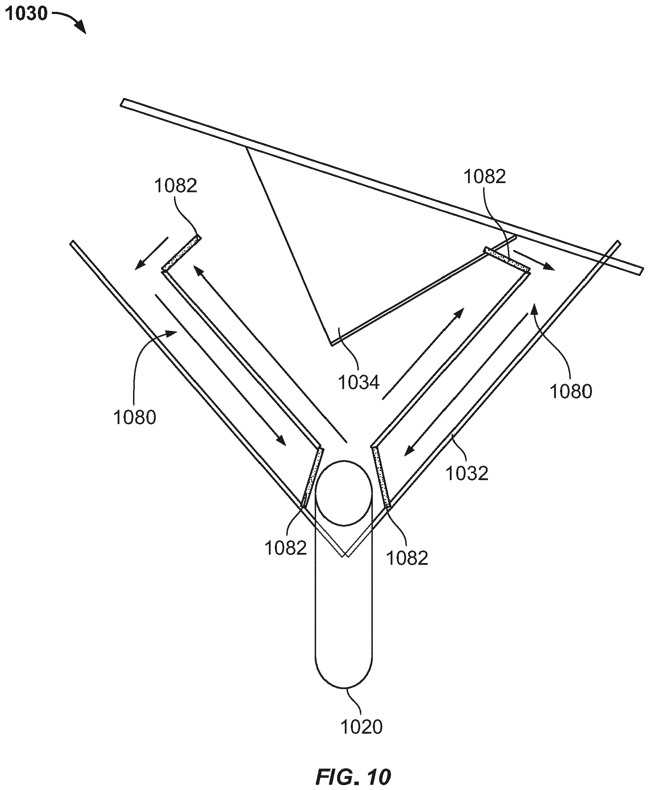

**FIG. 10** illustrates an embodiment of a fluid path valve in accordance with the present disclosure. As shown in FIG. 10, a fluid path valve 1030 may include a V-feature 1032 and a bistable mechanism 1034. V-feature 1032 may be associated with drop offs 1082 that may form paths or channels 1080. In some embodiments, drop offs 1082 may cause detent 1020 to only move in the direction indicated by the arrows (and in the opposite direction during a return process) in FIG. 10, thereby preventing detent 1020 from touching the inner wall (for instance, bistable mechanism 1034) and rotating it as detent 1020 moves toward the corner of V-feature 1032, for example, only contacting bistable mechanism 1034 when traveling toward bistable mechanism and not when traveling away from bistable mechanism 1034 toward corner of V-feature 1032. In some embodiments, the paths 1080 may slope up (for instance, out of the page of FIG. 10) in the direction of the arrows of FIG. 10. This embodiment may be used in the system shown in earlier figures.

While the present disclosure has been illustrated and described in detail in the drawings and foregoing description, the same is to be considered as illustrative and not restrictive in character, it being understood that only the certain embodiments have been shown and described.

It should be understood that while the use of words such as preferable, preferably, preferred or more preferred utilized in the description above indicate that the feature so described may be more desirable, it nonetheless may not be necessary and embodiments lacking the same may be contemplated as within the scope of the present disclosure, the scope being defined by the claims that follow. In reading the claims, it is intended that when words such as "a," "an," "at least one," or "at least one portion" are used there is no intention to limit the claim to only one item unless specifically stated to the contrary in the claim. When the language "at least a portion" and/or "a portion" is used the item can include a portion and/or the entire item unless specifically stated to the contrary.

## Claims

1. A fluid pump system (105) for a wearable fluid delivery device (160), comprising:
a pump chamber (344; 444; 644) to store a fluid;
a fluid path in fluid communication with the pump chamber and a reservoir (164; 212);
a fluid path valve (330; 430; 530; 630; 730; 830; 930; 1030) operative to control a state of the fluid path, the fluid path valve comprising a fixed portion (332; 432; 532; 632; 732; 832; 932; 1032) and a movable portion (334; 434; 534; 634; 734; 834; 934; 1034);
**characterized by** a shaft comprising a detent arm (320; 520; 620; 720; 820; 920; 1020) configured to move along the fixed portion responsive to a force imparted by an actuator (316; 616),
wherein the fixed portion comprises a V-feature having a substantially V-shape, and wherein the movable portion comprises a substantially triangular projection extending from the movable portion to deflect a force of the shaft to cause the movable portion to pivot in one step from a first position to a second position, and in another step from the second position to the first position, responsive to being engaged by the shaft to change the state of the fluid path.

2. The pump system of claim 1, the wearable fluid delivery device configured to deliver a medicament to a patient, the medicament comprising insulin.

3. The pump system of one of the preceding claims, the movable portion comprising a bistable mechanism.

4. The pump system of one of the preceding claims, the state of the fluid path comprising one of a fluid delivery state to deliver the fluid to the patient from the pump chamber or a fluid fill state to fill the pump chamber from a reservoir in fluid communication with the fluid path.

5. The pump system of one of the preceding claims, the actuator comprising a shape memory alloy.

6. The pump system of one of the preceding claims, further comprising a spring (318) configured to bias the shaft in a direction toward the movable portion in the absence of the force of the actuator.

7. The pump system of one of the preceding claims, the movable portion associated with a first magnet attracted to a portion of the fixed portion to maintain the movable portion in contact with the fixed portion on a closed side of the fluid path valve until the shaft engages the movable portion to pivot the movable portion.

8. The pump system of one of the preceding claims, the V-feature comprising at least one channel for shaft to travel during operation of the fluid path valve.

9. The pump system of claim 8, the at least on channel comprising a sloped channel with at least one drop-off element to prevent the shaft from engaging an inner wall of the movable portion when traveling toward a central V-section of the V-feature.

## Patentansprüche

1. Fluidpumpensystem (105) für eine tragbare Fluidfördervorrichtung (160), umfassend:
eine Pumpenkammer (344; 444; 644) zum Speichern eines Fluids;
einen Fluidweg in Fluidverbindung mit der Pumpenkammer und einem Reservoir (164; 212);
ein Fluidwegventil (330; 430; 530; 630; 730; 830; 930; 1030), das betriebsfähig ist, einen Zustand des Fluidwegs zu steuern, wobei das Fluidwegventil einen feststehenden Abschnitt (332; 432; 532; 632; 732; 832; 932; 1032) und einen bewegbaren Abschnitt (334; 434; 534; 634; 734; 834; 934; 1034) umfasst;
**gekennzeichnet durch** einen Schaft, der einen Arretierarm (320; 520; 620; 720; 820; 920; 1020) umfasst, der dazu ausgelegt ist, sich als Reaktion auf eine durch einen Aktuator (316; 616) ausgeübte Kraft entlang des feststehenden Abschnitts zu bewegen,
wobei der feststehende Abschnitt ein V-Merkmal mit einer im Wesentlichen V-Form umfasst, und wobei der bewegbare Abschnitt einen im Wesentlichen dreieckigen Vorsprung umfasst, der sich von dem bewegbaren Abschnitt erstreckt, um eine Kraft des Schafts abzulenken, um den bewegbaren Abschnitt als Reaktion darauf, dass er durch die Welle in Eingriff genommen wird, zu veranlassen, in einem Schritt aus einer ersten Stellung in eine zweite Stellung und in einem anderen Schritt aus der zweiten Stellung in die erste Stellung zu schwenken, um den Zustand des Fluidwegs zu ändern.

2. Pumpensystem nach Anspruch 1, wobei die tragbare Flüssigkeitsfördervorrichtung dazu ausgelegt ist, einem Patienten ein Medikament zuzuführen, wobei das Medikament Insulin umfasst.

3. Pumpensystem nach einem der vorhergehenden Ansprüche, wobei der bewegbare Abschnitt einen bistabilen Mechanismus umfasst.

4. Pumpensystem nach einem der vorhergehenden Ansprüche, wobei der Zustand des Fluidwegs einen von einem Fluidförderzustand, um dem Patienten das Fluid aus der Pumpenkammer zuzuführen, oder einem Fluidfüllzustand, um die Pumpenkammer aus einem mit dem Fluidweg in Fluidverbindung stehenden Reservoir zu füllen, umfasst.

5. Pumpensystem nach einem der vorhergehenden Ansprüche, wobei der Aktuator eine Formgedächtnislegierung umfasst.

6. Pumpensystem nach einem der vorhergehenden Ansprüche, ferner umfassend eine Feder (318) die dazu ausgelegt ist, den Schaft bei fehlender Kraft des Aktuators zum bewegbaren Abschnitt hin vorzuspannen.

7. Pumpensystem nach einem der vorhergehenden Ansprüche, wobei der bewegbare Abschnitt einem ersten Magneten zugeordnet ist, der von einem Abschnitt des feststehenden Abschnitts angezogen wird, um den bewegbaren Abschnitt in Kontakt mit dem feststehenden Abschnitt auf einer geschlossenen Seite des Fluidwegventils zu halten, bis der Schaft mit dem bewegbaren Abschnitt in Eingriff kommt, um den bewegbaren Abschnitt zu schwenken.

8. Pumpensystem nach einem der vorhergehenden Ansprüche, wobei das V-Merkmal mindestens einen Kanal umfasst, in dem sich der Schaft während des Betriebs des Fluidwegventils bewegen kann.

9. Pumpensystem nach Anspruch 8, wobei der mindestens eine Kanal einen geneigten Kanal mit mindestens einem abfallenden Element umfasst, um zu verhindern, dass der Schaft beim Bewegen in Richtung eines mittigen V-Abschnitts des V-Merkmals mit einer Innenwand des bewegbaren Abschnitts in Eingriff kommt.

## Revendications

1. Système de pompe à fluide (105) pour un dispositif portable d'administration de fluide (160), comprenant :
une chambre de pompe (344 ; 444 ; 644) destinée à stocker un fluide ;
un chemin de fluide en communication fluidique avec la chambre de pompe et un réservoir (164 ; 212) ;
une soupape de chemin de fluide (330 ; 430 ; 530 ; 630 ; 730 ; 830 ; 930 ; 1030) fonctionnelle pour commander un état du chemin de fluide, la soupape de chemin de fluide comprenant une partie fixe (332 ; 432 ; 532 ; 632 ; 732 ; 832 ; 932 ; 1032) et une partie mobile (334 ; 434 ; 534 ; 634 ; 734 ; 834 ; 934 ; 1034) ;
**caractérisé par** une tige comprenant un bras à encliquetage (320 ; 520 ; 620 ; 720 ; 820 ; 920 ; 1020) configuré pour se mouvoir le long de la partie fixe en réponse à une force transmise par un actionneur (316 ; 616),
dans lequel la partie fixe comprend un organe en V présentant une forme sensiblement de V, et dans lequel la partie mobile comprend une saillie sensiblement triangulaire s'étendant depuis la partie mobile pour dévier une force de la tige pour amener la partie mobile à pivoter dans une étape d'une première position à une seconde position, et dans une autre étape de la seconde position à la première position, en réponse au fait que la tige entre en prise avec celle-ci pour changer l'état du chemin de fluide.

2. Système de pompe de la revendication 1, le dispositif portable d'administration de fluide étant configuré pour administrer un médicament à un patient, le médicament comprenant de l'insuline.

3. Système de pompe de l'une des revendications précédentes, la partie mobile comprenant un mécanisme bistable.

4. Système de pompe de l'une des revendications précédentes, l'état du chemin de fluide comprenant un d'un état d'administration de fluide pour administrer le fluide au patient depuis la chambre de pompe ou un état de remplissage de fluide pour remplir la chambre de pompe depuis un réservoir en communication fluidique avec le chemin de fluide.

5. Système de pompe de l'une des revendications précédentes, l'actionneur comprenant un alliage à mémoire de forme.

6. Système de pompe de l'une des revendications précédentes, comprenant en outre un ressort (318) configuré pour solliciter la tige dans une direction vers la partie mobile en l'absence de la force de l'actionneur.

7. Système de pompe de l'une des revendications précédentes, la partie mobile étant associée à un premier aimant attiré par une partie de la partie fixe pour maintenir la partie mobile en contact avec la partie fixe sur un côté fermé de la soupape de chemin de fluide jusqu'à ce que la tige entre en prise avec la partie mobile pour faire pivoter la partie mobile.

8. Système de pompe de l'une des revendications précédentes, l'organe en V comprenant au moins une gorge pour le déplacement de la tige durant le fonctionnement de la soupape de chemin de fluide.

9. Système de pompe de la revendication 8, l'au moins une gorge comprenant une gorge en pente avec au moins un élément d'abaissement pour empêcher la tige d'entrer en prise avec une paroi intérieure de la partie mobile lors du déplacement vers une section en V centrale de l'organe en V.
